# FASCICULE DE BREVET EUROPEEN

(11) **EP 3 661 450 B1**
(45) Date de publication et mention de la délivrance du brevet: **28.02.2024**
(21) Numéro de dépôt: 18762574.4
(22) Date de dépôt: 19.07.2018
(51) Int. Cl.: A61B 90/00, A61B 17/24, A61B 5/00

(54) **IMPLANT A USAGE MÉDICAL DESTINÉ À SE CLIPPER A UNE PROTUBÉRANCE BIOLOGIQUE**
IMPLANTAT ZUR MEDIZINISCHEN VERWENDUNG ZUM KLEMMEN AUF EINE BIOLOGISCHE PROTUBERANZ
IMPLANT FOR MEDICAL USE INTENDED TO CLIP TO A BIOLOGICAL PROTUBERANCE

(30) Priorité: 03.08.2017 FR 1757468
(43) Date de publication de la demande: 10.06.2020
(73) Titulaire: Dianosic, 67000 Strasbourg (FR)
(72) Inventeur: AUGUSTIN, Marc, 75015 Paris (FR); BASTIDE, Philippe, 92130 Issy-les-Moulineaux (FR)
(74) Mandataire: Bonnet, Michel
(86) Numéro de dépôt international: PCT/FR2018/051848
(87) Numéro de publication internationale: WO 2019/025695

(56) Documents cités:
- DE-U1- 20 314 392
- US-A- 5 094 233
- US-A1- 2013 338 700
- US-A1- 2016 374 800

## Description

La présente invention concerne un implant à usage médical destiné à se clipper à une protubérance biologique.

L'invention s'applique plus particulièrement à un implant à usage médical destiné à être introduit dans une cavité de corps humain ou animal, comportant au moins un clip comprenant deux parois destinées à enserrer entre elles une protubérance biologique présente dans la cavité afin de fixer l'implant à la protubérance biologique.

La demande de brevet américain publiée sous le numéro US 2013/0276794 A1 décrit un tel implant. Il est destiné à protéger le cornet moyen pendant une opération chirurgicale dans la cavité nasale. Ainsi, l'utilisation de l'implant n'est nécessaire que pour le temps de l'opération chirurgicale. Il n'est donc pas adapté à tenir longtemps en place, par exemple plusieurs mois. US2013/0338700 aussi décrit un implant selon l'état de la technique.

Il peut ainsi être souhaité de prévoir un implant à usage médical qui permette de s'affranchir d'au moins une partie des problèmes et contraintes précités.

L'invention a donc pour objet un implant à usage médical destiné à être introduit dans une cavité de corps humain ou animal, comportant au moins un clip comprenant deux parois destinées à enserrer entre elles une protubérance biologique présente dans la cavité afin de fixer l'implant à la protubérance biologique, l'implant étant caractérisé en ce que chaque paroi comporte un élément de structure présentant des ondulations de sorte que l'élément de structure soit replié plusieurs fois sur lui-même, permettant à la paroi d'être repliée avant l'introduction de l'implant, puis déployée à l'intérieur de la cavité.

Ainsi, l'implant selon l'invention peut présenter de grandes parois, lui permettant d'être fixé de manière stable et durable à la protubérance biologique, tout en pouvant être introduit dans la cavité au travers d'une ouverture étroite.

De façon optionnelle, l'élément de structure est une tige.

De façon optionnelle également, l'implant a une forme allongée selon une direction longitudinale et comporte en outre au moins un stabilisateur dépassant des parois selon une direction perpendiculaire à la direction longitudinale.

De façon optionnelle également, l'implant comporte une partie avant comportant un stabilisateur avant, une partie centrale comportant les parois et une partie arrière comportant un stabilisateur arrière.

De façon optionnelle également, le stabilisateur comporte une structure maillée conçue pour être aplatie avant l'introduction de l'implant, puis déployée à l'intérieur de la cavité.

De façon optionnelle également, la structure maillée présente des mailles ayant chacune une surface d'au moins 0,1 cm².

De façon optionnelle également, l'implant présente en outre un revêtement anti-inflammatoire et/ou antimicrobien.

De façon optionnelle également, au moins l'élément de structure comporte un matériau à mémoire de forme conçu pour se déployer au-delà d'une température prédéfinie.

De façon optionnelle également, au moins l'élément de structure comporte un matériau superélastique et/ou résorbable.

De façon optionnelle également, chaque paroi d'au moins un du ou des clips présente une hauteur diminuant d'un bord arrière jusqu'à un bord avant de la paroi.

De façon optionnelle également, chaque paroi d'au moins un du ou des clips présente une hauteur constante d'un bord arrière jusqu'à un bord avant de la paroi.

De façon optionnelle également, l'implant comporte en outre, pour chaque paroi d'au moins un du ou des clips, un élément de renfort structurel s'étendant le long d'un bord de cette paroi.

De façon optionnelle également, chaque élément de renfort structurel comporte une tige présentant des ondulations permettant une compression de cet élément de renfort structurel.

De façon optionnelle également, le ou les clips comprennent deux clips destinés à enserrer respectivement deux protubérances biologiques présentes dans la cavité afin de fixer l'implant aux deux protubérances biologiques à la fois.

De façon optionnelle également, les deux clips sont situés l'un au-dessus de l'autre de manière à pouvoir enserrer respectivement un cornet moyen et un cornet inférieur d'une cavité nasale.

L'invention sera mieux comprise à l'aide de la description qui va suivre, donnée uniquement à titre d'exemple et faite en se référant aux dessins annexés dans lesquels :
- les figures 1 à 4 représentent schématiquement la structure générale d'un implant à usage médical selon un premier mode de réalisation de l'invention, à l'état déployé,
- la figure 5 représente schématiquement une cavité nasale d'un être humain,
- la figure 6 représente schématiquement l'implant à l'état replié,
- la figure 7 représente schématiquement un dispositif de déploiement de l'implant,
- la figure 8 représente schématiquement l'implant clippé sur une protubérance biologique,
- la figure 9 représente schématiquement la structure générale d'un implant à usage médical, à l'état déployé, selon un deuxième mode de réalisation de l'invention, et
- la figure 10 représente schématiquement un état comprimé de l'implant de la figure 9.

Dans la description qui va suivre, les éléments seront décrits en référence à un repère orthogonal formé d'une direction longitudinale L, d'une direction verticale V et d'une direction transversale T.

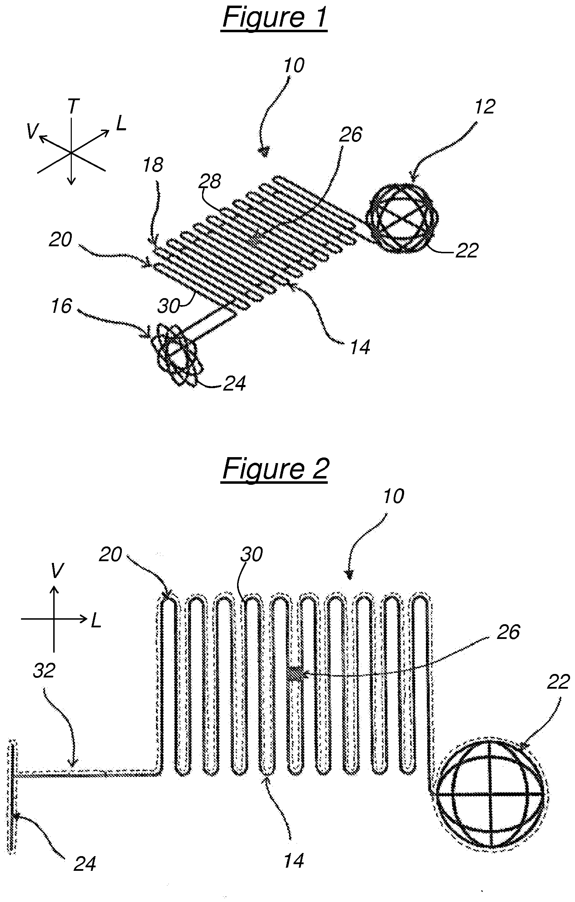

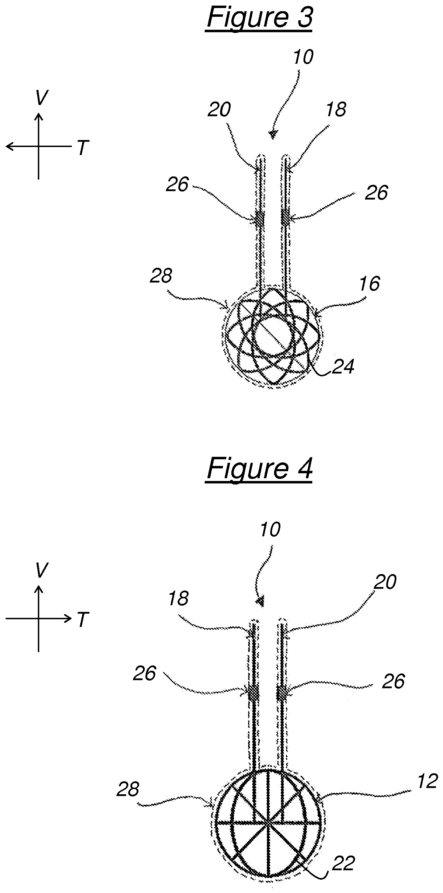

L'implant 10 à usage médical selon un premier mode de réalisation de l'invention, représenté schématiquement sur les figures 1 à 4 à l'état déployé est de forme générale allongée selon la direction longitudinale L et présente une partie avant 16, une partie centrale 14 et une partie arrière 12.

La partie centrale 14 est destinée à se clipper sur une protubérance biologique présente dans une cavité de corps humain ou animal. Pour cela, la partie centrale 14 comporte deux parois 18, 20 qui s'étendent sensiblement l'une en face de l'autre et qui sont destinées à enserrer entre elles la protubérance biologique. Chaque paroi 18, 20 présente un bord inférieur fixé à la partie avant 16 et à la partie arrière 12 de l'implant 10. Dans l'exemple décrit, les deux parois 18, 20 sont planes et s'étendent parallèlement au plan défini par la direction longitudinale et la direction verticale. En outre, les parois 18, 20 ont une longueur comprise entre 30 et 50 mm, par exemple 40 mm, et une hauteur comprise entre 20 et 30 mm, par exemple 25 mm.

Par ailleurs, dans l'exemple décrit, la partie avant 16 et la partie arrière 12 comportent respectivement un stabilisateur avant 24 et un stabilisateur arrière 22 destinés à stabiliser l'implant 10 dans la cavité. Pour cela, chacun des stabilisateurs 22, 24 dépasse verticalement et/ou transversalement de la partie centrale 14, de sorte que l'implant 10 présente un encombrement vertical et/ou transversal supérieur à celui de la partie centrale 14 seule, et en particulier des parois 18, 20 seules. Ainsi, l'implant 10 est peu susceptible de bouger dans la cavité, car son encombrement le fait rapidement venir au contact des parois de la cavité. En outre, avec cet encombrement, l'implant 10 est peu susceptible de passer par une ouverture menant à la cavité et court donc peu de risque de sortir de la cavité. Dans l'exemple décrit, le stabilisateur arrière 22 est en forme de disque et le stabilisateur avant 24 est en forme de sphère. En outre, les stabilisateurs 22, 24 ont un diamètre compris entre 10 et 20 mm, par exemple 15 mm. De manière générale, la forme et la taille des stabilisateurs 22, 24 sont adaptées à la forme de la cavité.

L'implant 10 peut en outre comporter au moins un capteur 26 d'une grandeur biologique. Chaque capteur est par exemple fixé sur la partie centrale 14 de l'implant 10 et permet l'évaluation in situ d'au moins un paramètre biologique tel que, sans que cela soit restrictif, le taux d'oxygénation de tissus biologiques ou bien la pression avoisinant l'implant 10. De préférence, chaque capteur 26 comporte un système de communication sans fil permettant de communiquer les données recueillies par le capteur 26 en direction d'un dispositif informatique, tel qu'un téléphone intelligent (« smartphone » en anglais) ou une tablette, situé à l'extérieur du corps humain ou animal. La présence du ou des capteurs 26 sur l'implant 10 permet ainsi la surveillance à distance de différents paramètres biologiques, dont la connaissance est utile en otorhinolaryngologie, neurochirurgie ou toute autre spécialité médicale.

Un logiciel dédié peut être programmé pour détecter une déviation des mesures par rapport à un intervalle normal dans lequel elles devraient se trouver. En cas de déviation détectée, une alerte automatique peut être envoyée (par exemple par courriel ou par message téléphonique court (SMS)) au professionnel de santé pour qu'il prévienne par exemple le patient chez qui l'implant 10 est posé. Alternativement ou bien en complément, l'alerte automatique peut être directement envoyée au patient, ou bien encore à un centre de traitement d'appels qui se chargera d'appeler le professionnel de santé et/ou le patient. Une fois les mesures obtenues, elles peuvent être utilisées par le professionnel de santé en vue de réagir à un évènement potentiellement non prévu une fois le patient sorti de l'hôpital, ou de poser ou d'affiner son diagnostic permettant ainsi de personnaliser le traitement du patient. Par ailleurs, les mesures obtenues peuvent être utilisées pour constituer ou bien alimenter des registres ou tout autre type de base de données.

Pour faciliter l'introduction de l'implant 10 dans la cavité, chaque paroi 18, 20 comporte une tige 28, 30 présentant des ondulations longitudinales de sorte que la tige 28, 30 soit repliée plusieurs fois sur elle-même. Plus précisément, la tige présente des barreaux parallèles verticaux reliés à leurs extrémités par des méandres alternant d'une extrémité à l'autre des barreaux. Ainsi, chaque paroi 18, 20 peut être repliée longitudinalement en rapprochant les barreaux les uns des autres. Par ailleurs, la tige 28, 30 présente une extrémité avant fixée au stabilisateur avant 24 et une extrémité arrière fixée au stabilisateur arrière 22.

Toujours pour faciliter l'introduction de l'implant 10, les stabilisateurs 22, 24 comportent une structure maillée conçue pour être aplatie avant l'introduction de l'implant 10. De préférence, la structure maillée comporte des mailles ayant chacune une surface d'au moins 0,1 cm² afin de permettre l'écoulement de fluides biologiques au travers des stabilisateurs 22, 24.

De préférence, les tiges des parois 18, 20 et les structures maillées des stabilisateurs 22, 24 sont formées dans un matériau présentant des propriétés de mémoire de forme et/ou de superélasticité.

La propriété de mémoire de forme permet au matériau ayant été déformé de récupérer, lorsqu'il est chauffé au-dessus d'une température prédéfinie, sa forme initiale. Par exemple, le matériau est conçu pour retourner automatiquement à sa forme déployée lorsque sa température dépasse un seuil prédéfini, ce seuil étant de préférence compris entre 36°C et 38°C, par exemple 37°C. Ces températures correspondent aux températures usuelles dans les cavités d'un corps humain ou animal.

La propriété d'élasticité permet au matériau ayant été déformé par une contrainte de récupérer, lorsque la contrainte cesse, sa forme initiale (on parle de déformation élastique). La propriété de superélasticité est identique mais s'étend à des déformations très importantes.

De préférence, un matériau dit « à mémoire de forme » (en anglais : « shape memory alloy ») est utilisé, car les matériaux à mémoire de forme présentent les deux propriétés de mémoire de forme et de superélasticité. Par exemple, le matériau à mémoire de forme utilisé est du Nitinol, un alliage nickel-titane de plus en plus utilisé dans le domaine médical, du fait de ses capacités superélastiques, sa mémoire de forme et sa bonne tolérance par l'organisme.

En outre, de préférence, les tiges 28, 30 des parois 18, 20 et les maillages des stabilisateurs 22, 24 sont réalisés à partir d'un seul fil, plié pour obtenir les formes voulues, de préférence sans soudure.

En référence aux figures 2 à 4, l'implant 10 présente de préférence un revêtement anti-inflammatoire et/ou antimicrobien (représenté sur les figures par les pointillés 32). Il peut en outre présenter un revêtement thérapeutique, par exemple anti-infectieux, anti-cancéreux, anti-granulome, corticoïde ou de traitement de la polypose ou de la sinusite chronique. Chaque revêtement est apte à fournir le traitement associé pendant une durée prédéfinie, par exemple au moins une semaine.

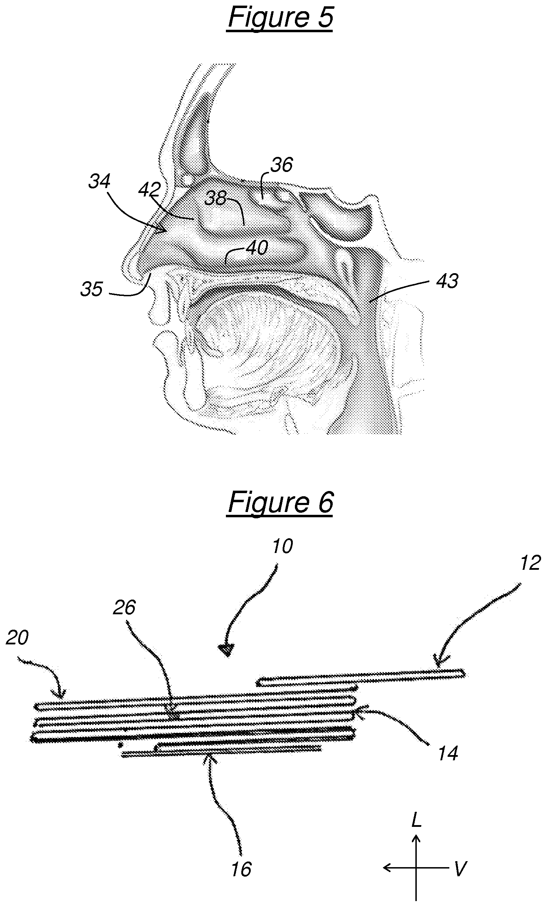

Dans l'exemple décrit, l'implant 10 est destiné à être introduit dans une cavité nasale 34 représentée sur la figure 5. La cavité nasale 34 (également appelée fosse nasale) s'étend en arrière de l'orifice narinaire 35. Les cornets supérieur 36, moyen 38 et inférieur 40 jouent un rôle de filtre et permettent une bonne circulation de l'air et autres fluides. Le processus unciforme 42, situé latéralement par rapport au cornet moyen 38 délimite l'entrée de l'ostium du sinus maxillaire.

Suite à une chirurgie affectant le cornet moyen 38 et/ou le processus unciforme 42, l'intégrité de la muqueuse de la cavité nasale 34 peut être affectée. Il existe donc un risque que le cornet moyen 38 se latéralise, c'est-à-dire qu'il adhère au processus unciforme et/ou au cornet inférieur 40 et entraîne une cicatrisation non souhaitée. Dans l'exemple décrit, l'implant 10 vise à réduire le risque de latéralisation du cornet moyen 38.

En référence à la figure 6, l'implant 10 est illustré dans un état replié dans lequel les parois 18, 20 sont repliées le long de la direction longitudinale L et les stabilisateurs 22, 24 sont aplatis et redressés dans le plan défini par la direction verticale V et la direction transversale T. Ainsi, l'implant 10 est très compact et présente une forme allongée le long de la direction verticale V.

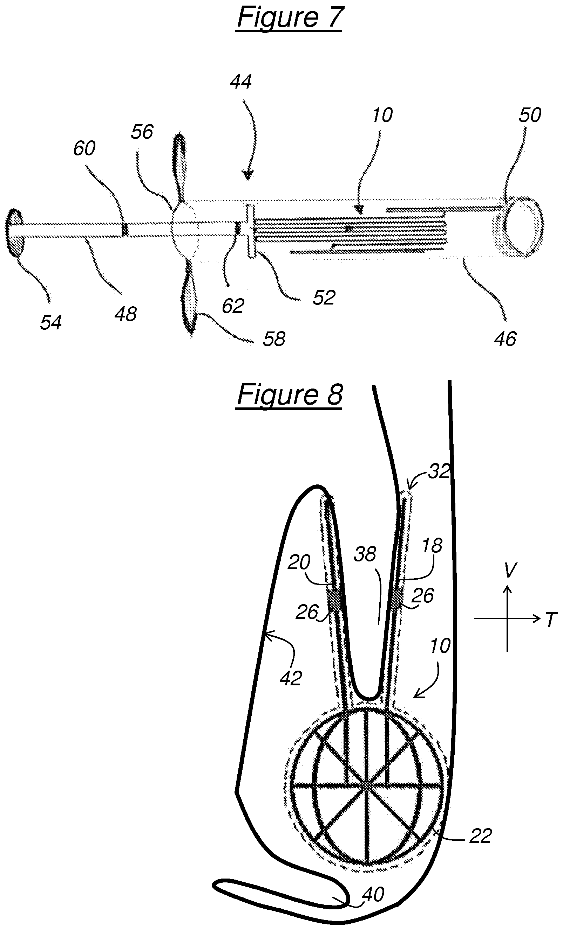

Un dispositif de déploiement 44 illustré sur la figure 7 peut être utilisé pour déployer l'implant 10 dans la cavité nasale 34.

Le dispositif de déploiement 44 présente la forme générale d'une seringue avec un tube creux 46, de préférence transparent, dans lequel l'implant 10 à l'état replié est destiné à être placé et un piston 48 coulissant dans le tube creux 46 pour pousser l'implant 10 vers une extrémité distale 50 du tube creux 46. De préférence, l'extrémité distale 50 est composée d'une matière plus souple que le reste du tube creux 46 afin de ne pas endommager les muqueuses lors de son introduction et renforcer ainsi le confort du patient.

Le piston 48 est rigide et présente, à une extrémité distale, un plateau 52 et, à une extrémité proximale, un bouton poussoir 54. Le dispositif de déploiement 44 comporte en outre, au niveau d'une extrémité proximale 56 du tube creux 46, des ailettes de rétraction 58 permettant, en coopération avec le bouton poussoir 54, à un utilisateur de faire manuellement coulisser le piston 48 par rapport au tube creux 46.

Le piston 48 est muni d'un marqueur médial 60 et d'un marqueur distal 62. Le marqueur distal 62 est séparé du bouton poussoir 54 d'une distance correspondant à la longueur (le long de la direction vertical V) de l'implant 10 à l'état replié. Les marqueurs 60, 62 sont destinés à s'assurer du bon positionnement de l'implant 10 dans la cavité nasale 34, comme cela sera expliqué par la suite.

Pour introduire l'implant 10 dans la cavité nasale 34, le tube creux 46 contenant l'implant 10 est inséré dans la narine 35 puis dans la cavité nasale 34 jusqu'à amener son extrémité proximale 56 au niveau de l'entrée de la narine 35. Le piston 48 est ensuite inséré dans le tube creux 46 par son extrémité proximale 56, le plateau 52 venant au contact de l'implant 10. Par action simultanée sur le bouton poussoir 54 et les ailettes de rétraction 58, le tube creux 46 est alors extrait progressivement de la cavité nasale 34 alors que le piston 48 expulse l'implant 10 dans la cavité nasale 34 par l'extrémité distale 50 du tube creux 46, à proximité du cornet moyen 38.

Les marqueurs 60, 62 permettent de se repérer pendant cette phase. Plus précisément, l'alignement du marqueur distal 62 avec l'extrémité proximale 56 du tube creux 46 indique que l'implant 10 est sur le point de commencer à sortir de l'extrémité distale 50 du tube creux 46. En outre, l'alignement du marqueur médial 60 avec l'extrémité proximale 56 du tube creux 46 indique que la moitié de l'implant 10 est sorti du tube creux 46 par son extrémité distale 50. Enfin, le contact entre le bouton poussoir 54 et l'extrémité proximale 56 du tube creux 46 indique que la totalité de l'implant 10 est sorti du tube creux 46.

La température dans la cavité nasale 34 étant d'environ 37° C, l'implant 10 à mémoire de forme est chauffé par la cavité nasale 34 à 37 °C, ce qui est supérieur au seuil de déploiement de sorte que l'implant 10 se déploie, c'est-à-dire qu'il reprend automatiquement la configuration illustrée sur les figures 1 à 4. Alternativement, lorsque la propriété de superélasticité est utilisée, l'implant 10 reprend automatiquement sa forme initiale en sortant du tube creux 46, puisque la contrainte exercée par ce dernier sur l'implant 10 cesse. Dans un cas comme dans l'autre, les deux parois 18, 20 se déploient de part et d'autre du cornet moyen 38 de manière à l'enserrer comme cela est illustré sur la figure 8. Ainsi, l'implant 10 est clippé sur le cornet moyen 38 et solidement amarré dans la cavité nasale 34. En outre, la présence des stabilisateurs 22, 24 empêche son expulsion par la narine 35, ou bien son ingestion par l'oropharynx 43, comme illustré sur la figure 5. Dans cette position, l'implant 10 forme une barrière physique pour empêcher le cornet moyen 38 de venir au contact du processus unciforme 42 et/ou du cornet inférieur 40. En outre, le revêtement anti-inflammatoire et/ou antimicrobien 32 permet une cicatrisation rapide des tissus à vif et diminue donc le risque de latéralisation du cornet moyen 38. Par ailleurs, le revêtement 32 peut aussi permettre, sans qu'une intervention chirurgicale n'ait nécessairement été réalisée, de traiter la polypose, la sinusite ou toute autre affection de la fosse nasale que le revêtement 32 est conçu pour traiter. Enfin, le maillage des stabilisateurs 22, 24 permet le lavage de la cavité nasale 34.

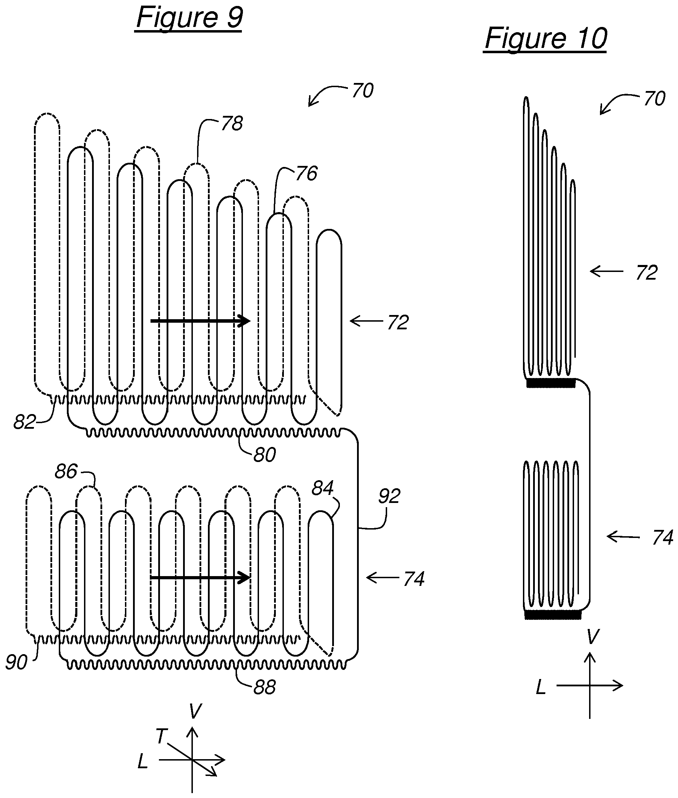

Un implant 70 à usage médical selon un deuxième mode de réalisation de l'invention, est représenté schématiquement à l'état déployé sur la figure 9. L'implant 70 comporte deux parties 72, 74 destinées à se clipper respectivement à deux protubérances biologiques présentes dans une cavité de corps humain ou animal. Dans l'exemple décrit, les deux parties 72, 74 sont superposées verticalement l'une à l'autre. Par exemple, la partie supérieure 72 est destinée à se clipper au cornet moyen 38, tandis que la partie inférieure 74 est destinée à se clipper au cornet inférieur 40.

Pour cela, chaque partie 72, 74 est réalisée d'une manière similaire à la partie centrale 14 de l'implant 10 selon le premier mode de réalisation de l'invention.

Ainsi, la partie supérieure 72 comporte deux parois, respectivement définies par deux tiges 76, 78 présentant des ondulations longitudinales, qui s'étendent sensiblement l'une en face de l'autre et qui sont destinées à enserrer entre elles la première protubérance biologique. La paroi située en arrière-plan sur la figure 9 est représentée en traits pointillés pour la distinguer de la paroi située au premier plan. Dans l'exemple décrit, les deux parois sont planes et s'étendent parallèlement au plan défini par la direction longitudinale L et la direction verticale V. Par exemple, les parois ont une longueur comprise entre 30 et 50 mm, par exemple 40 mm, et une hauteur comprise entre 20 et 30 mm, par exemple 25 mm. Du fait des ondulations longitudinales, chaque tige 76, 78 est repliée plusieurs fois sur elle-même. Plus précisément, chaque tige 76, 78 présente des barreaux parallèles verticaux reliés à leurs extrémités par des méandres alternant d'une extrémité à l'autre des barreaux. Ainsi, chaque paroi peut être repliée longitudinalement en rapprochant les barreaux les uns des autres. Dans l'exemple décrit, ces barreaux présentent des longueurs diminuant de l'arrière vers l'avant, de sorte que chaque paroi présente une hauteur diminuant d'un bord arrière jusqu'à un bord avant de la paroi.

La partie supérieure 72 comporte en outre, pour chaque paroi, un élément de renfort structurel s'étendant longitudinalement le long d'un bord inférieur de la paroi. Dans l'exemple décrit, chaque élément de renfort structurel comporte une tige 80, 82 respective présentant des ondulations longitudinales afin de permettre la compression longitudinale de l'élément de renfort structurel en même temps que celle de la paroi associée. Les ondulations longitudinales des tiges 80, 82 présentent une hauteur beaucoup plus faible que les ondulations des tiges 76, 78, par exemple au moins dix fois plus petite.

De même, la partie inférieure 74 comporte deux parois définies par respectivement deux tiges 84, 86 présentant des ondulations longitudinales. Dans l'exemple décrit, les parois de la partie inférieure 74 présentent une hauteur constante, par exemple comprise entre 10 et 30 mm. La partie inférieure 74 comporte en outre deux éléments de renfort structurel s'étendant longitudinalement le long de bords inférieurs respectivement des parois. Ces éléments de renfort structurel comportent dans l'exemple décrit respectivement deux tiges 88, 90 présentant des ondulations longitudinales, comme pour la partie supérieure 72, ces ondulations longitudinales ayant une hauteur beaucoup plus faible que celle des ondulations longitudinales des tiges 86, 84, par exemple au moins dix fois plus petite.

L'implant 70 comporte en outre un élément de liaison attachant la partie inférieur 74 à la partie supérieure 72. Dans l'exemple décrit, cet élément de liaison comporte une tige 92 sensiblement verticale connectant des extrémités avant de deux éléments de renfort structurel respectivement des parties supérieure 72 et inférieure 74.

Comme pour l'implant 10 selon le premier mode de réalisation, les tiges 76 - 92 sont par exemple formées dans un matériau présentant des propriétés de mémoire de forme et/ou de superélasticité.

En outre, de préférence, les tiges 76 - 92 sont réalisées à partir d'un seul fil, plié pour obtenir les formes voulues, de préférence sans soudure.

En outre, de préférence, les tiges 76 - 92 sont réalisées dans un matériau résorbable, c'est-à-dire que le matériau est conçu pour disparaître complètement par absorption progressive sous l'effet de l'environnement biologique de la cavité, par exemple en moins de deux ans. De préférence, lorsque qu'un matériau résorbable est utilisé, aucune soudure n'est prévue.

Il sera en outre apprécié que, dans l'exemple décrit, l'implant 70 est dénué de capteur.

Ainsi, les parois définies par les tiges 76, 78, 84, 86, ainsi que les éléments de renfort structurel formés par les tiges 80, 82, 88, 90, peuvent être repliés sur eux-mêmes longitudinalement, comme indiqué par les deux flèches en gras sur la figure 9, pour obtenir l'état comprimé illustré sur la figure 10. Dans cet état comprimé, l'implant 70 peut être introduit dans la cavité nasale 34 pour y être déployé en revenant à l'état déployé de la figure 9, avec le clip formé par la partie supérieure 72 enserrant le cornet moyen 38 et le clip formé par la partie inférieure 74 enserrant le cornet inférieur 40.

Il apparaît clairement qu'un implant à usage médical tel que ceux décrits précédemment est apte à se clipper de manière durable sur une protubérance biologique, tout en pouvant être facilement introduit à l'état replié dans la cavité. Il est particulièrement adapté à des interventions en chirurgie otorhinolaryngologie, voire en neurochirurgie, ou dans tout autre type de spécialité chirurgicale nécessitant le recours à ce type d'implant. Dans ce cas, les formes de l'implant 10 ou 70 peuvent être adaptées à toutes les applications souhaitées, et peuvent donc être très diverses.

On notera par ailleurs que l'invention n'est pas limitée aux modes de réalisation décrits précédemment. Il apparaîtra en effet à l'homme de l'art que diverses modifications peuvent être apportées au mode de réalisation décrit ci-dessus, à la lumière de l'enseignement qui vient de lui être divulgué.

En particulier, chaque paroi pourrait comporter, à la place de la tige, une feuille pliée en accordéon.

Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

## Revendications

1. Implant (10 ; 70) à usage médical destiné à être introduit dans une cavité (34) de corps humain ou animal, comportant au moins un clip comprenant deux parois (18, 20) destinées à enserrer entre elles une protubérance biologique (38) présente dans la cavité (34) afin de fixer l'implant (10) à la protubérance biologique (38), l'implant (10) étant **caractérisé en ce que** chaque paroi (18, 20) comporte un élément de structure (28, 30 ; 76, 78 ; 84, 86) présentant des ondulations de sorte que l'élément de structure (28, 30 ; 76, 78 ; 84, 86) soit replié plusieurs fois sur lui-même, permettant à la paroi (18, 20) d'être repliée avant l'introduction de l'implant (10 ; 70), puis déployée à l'intérieur de la cavité (34).

2. Implant (10; 70) selon la revendication 1, dans lequel l'élément de structure (28, 30 ; 76, 78 ; 84, 86) est une tige.

3. Implant (10) selon la revendication 1 ou 2, ayant une forme allongée selon une direction longitudinale (L) et comportant en outre au moins un stabilisateur (22, 24) dépassant des parois (18, 20) selon une direction perpendiculaire (V, T) à la direction longitudinale (L).

4. Implant (10) selon la revendication 3, comportant une partie avant (16) comportant un stabilisateur avant (24), une partie centrale (14) comportant les parois (18, 20) et une partie arrière (12) comportant un stabilisateur arrière (22).

5. Implant (10) selon la revendication 3 ou 4, dans lequel le stabilisateur (22, 24) comporte une structure maillée conçue pour être aplatie avant l'introduction de l'implant (10), puis déployée à l'intérieur de la cavité (34).

6. Implant (10) selon l'une quelconque des revendications 1 à 5, dans lequel les deux parois (18, 20) sont planes et s'étendent parallèlement sensiblement l'une en face de l'autre pour l'enserrement entre elles de la protubérance biologique (38).

7. Implant (10 ; 70) selon l'une quelconque des revendications 1 à 6, présentant en outre un revêtement anti-inflammatoire et/ou antimicrobien (32).

8. Implant (10 ; 70) selon l'une quelconque des revendications 1 à 7, dans lequel au moins l'élément de structure (28, 30 ; 76, 78 ; 84, 86) comporte un matériau à mémoire de forme conçu pour se déployer au-delà d'une température prédéfinie.

9. Implant (10 ; 70) selon l'une quelconque des revendications 1 à 8, dans lequel au moins l'élément de structure (28, 30 ; 76, 78 ; 84, 86) comporte un matériau superélastique et/ou résorbable.

10. Implant (70) selon l'une quelconque des revendications 1 à 9, dans lequel chaque paroi d'au moins un du ou des clips présente une hauteur diminuant d'un bord arrière jusqu'à un bord avant de la paroi.

11. Implant (10 ; 70) selon l'une quelconque des revendications 1 à 10, dans lequel chaque paroi d'au moins un du ou des clips présente une hauteur constante d'un bord arrière jusqu'à un bord avant de la paroi.

12. Implant (70) selon l'une quelconque des revendications 1 à 11, comportant en outre, pour chaque paroi d'au moins un du ou des clips, un élément de renfort structurel s'étendant le long d'un bord de cette paroi.

13. Implant (70) selon la revendication 12, dans lequel chaque élément de renfort structurel comporte une tige (80, 82, 88, 90) présentant des ondulations permettant une compression de cet élément de renfort structurel.

14. Implant (70) selon l'une quelconque des revendications 1 à 13, dans lequel le ou les clips comprennent deux clips destinés à enserrer respectivement deux protubérances biologiques (38, 40) présentes dans la cavité (34) afin de fixer l'implant (70) aux deux protubérances biologiques à la fois.

15. Implant (70) selon la revendication 14, dans lequel les deux clips sont situés l'un au-dessus de l'autre de manière à pouvoir enserrer respectivement un cornet moyen (38) et un cornet inférieur (40) d'une cavité nasale (34).

## Patentansprüche

1. Implantat (10; 70) zur medizinischen Anwendung, das bestimmt ist, um in einem Hohlraum (34) eines menschlichen oder tierischen Körpers untergebracht zu werden, und das mindestens einen Clip beinhaltet, der zwei Wände (18, 20) umfasst, die bestimmt sind, um eine biologische Protuberanz (38) zu umschließen, die im Hohlraum (34) vorhanden ist, um das Implantat (10) an der biologischen Protuberanz (38) zu fixieren, wobei das Implantat (10) **dadurch gekennzeichnet ist, dass** jede Wand (18, 20) ein Strukturelement (28, 30; 76, 78; 84, 86) beinhaltet, das Wellen aufweist, so dass das Strukturelement (28, 30; 76, 78; 84, 86) mehrmals auf sich selbst gefaltet wird, so dass die Wand (18, 20) vor dem Einführen des Implantats (10; 70) gefaltet und dann innerhalb des Hohlraums (34) entfaltet werden kann.

2. Implantat (10; 70) nach Anspruch 1, wobei das Strukturelement (28, 30; 76, 78; 84, 86) ein Stab ist.

3. Implantat (10) nach Anspruch 1 oder 2, das eine längliche Form entlang einer Längsrichtung (L) hat, und ferner mindestens einen Stabilisator (22, 24) beinhaltet, der über die Wände (18, 20) entlang einer Richtung hinausragt, die perpendikular (V, T) zur Längsrichtung (L) ist.

4. Implantat (10) nach Anspruch 3, einen vorderen Teil (16) beinhaltend, der einen vorderen Stabilisator (24) beinhaltet, einen mittleren Teil (14), der die Wände (18, 20) beinhaltet, und einem hinteren Teil (12), der einen hinteren Stabilisator (22) beinhaltet.

5. Implantat (10) nach Anspruch 3 oder 4, wobei der Stabilisator (22, 24) eine Maschenstruktur beinhaltend, die ausgelegt ist, um vor dem Einführen des Implantats (10) abgeflacht zu werden und dann innerhalb des Hohlraums (34) entfaltet zu werden.

6. Implantat (10) nach einem der Ansprüche 1 bis 5, wobei die zwei Wände (18, 20) eben sind und sich im Wesentlichen parallel zueinander erstrecken, um die biologische Protuberanz (38) zu umschließen.

7. Implantat (10; 70) nach einem der Ansprüche 1 bis 6, ferner eine entzündungshemmende und/oder antimikrobielle Beschichtung (32) aufweisend.

8. Implantat (10; 70) nach einem der Ansprüche 1 bis 7, wobei zumindest das Strukturelement (28, 30; 76, 78; 84, 86) ein Formgedächtnismaterial beinhaltet, das ausgelegt ist, um sich über einer vordefinierten Temperatur zu entfalten.

9. Implantat (10; 70) nach einem der Ansprüche 1 bis 8, wobei zumindest das Strukturelement (28, 30; 76, 78; 84, 86) ein superelastisches und/oder resorbierbares Material beinhaltet.

10. Implantat (70) nach einem der Ansprüche 1 bis 9, wobei jede Wand von mindestens einem des oder der Clips eine Höhe aufweist, die von einer hinteren Kante zu einer vorderen Kante der Wand abnimmt.

11. Implantat (10; 70) nach einem der Ansprüche 1 bis 10, wobei jede Wand von mindestens einem des oder der Clips eine Höhe aufweist, die von einer hinteren Kante bis zu einer vorderen Kante der Wand konstant ist.

12. Implantat (70) nach einem der Ansprüche 1 bis 11, das ferner für jede Wand von mindestens einem des oder der Clips ein Element der strukturellen Verstärkung beinhaltet, das sich entlang einer Kante dieser Wand erstreckt.

13. Implantat (70) nach Anspruch 12, wobei jedes Element der strukturellen Verstärkung einen Stab (80, 82, 88, 90) beinhaltet, der Wellen aufweist, die eine Kompression dieses Elements der strukturellen Verstärkung ermöglichen.

14. Implantat (70) nach einem der Ansprüche 1 bis 13, wobei der oder die Clips zwei Clips umfassen, die bestimmt sind, um jeweils zwei biologische Protuberanzen (38, 40), die im Hohlraum (34) vorhanden sind, zu umschließen, um das Implantat (70) gleichzeitig an den zwei biologischen Protuberanzen zu fixieren.

15. Implantat (70) nach Anspruch 14, wobei die zwei Clips so übereinander angeordnet sind, dass sie jeweils eine mittlere Muschel (38) und eine untere Muschel (40) einer Nasenhöhle (34) umschließen können.

## Claims

1. An implant (10; 70) for medical use intended to be introduced into a cavity (34) of a human or animal body, including at least one clip comprising two walls (18, 20) intended to grip therebetween a biological protuberance (38) present in the cavity (34) in order to attach the implant (10) to the biological protuberance (38), the implant (10) being **characterized in that** each wall (18, 20) includes a structure element (28 , 30; 76, 78; 84, 86) having undulations so that the structure element (28, 30; 76, 78; 84, 86) is folded up several times on itself, allowing the wall (18 , 20) to be folded up before the introduction of the implant (10; 70), then deployed inside the cavity (34).

2. The implant (10; 70) according to claim 1, wherein the structure element (28, 30; 76, 78; 84, 86) is a rod.

3. The implant (10) according to claim 1 or 2, having an elongated shape in a longitudinal direction (L) and further including at least one stabilizer (22, 24) protruding from the walls (18, 20) in a direction perpendicular (V, T) to the longitudinal direction (L).

4. The implant (10) according to claim 3, including a front part (16) including a front stabilizer (24), a central part (14) including the walls (18, 20) and a rear part (12) including a rear stabilizer (22).

5. The implant (10) according to claim 3 or 4, wherein the stabilizer (22, 24) includes a meshed structure designed to be flattened before the introduction of the implant (10), then deployed inside the cavity (34).

6. The implant (10) according to any one of claims 1 to 5, wherein the two walls (18, 20) are planar and extend substantially parallel facing each other to grip the biological protuberance (38) between them.

7. The implant (10; 70) according to any one of claims 1 to 6, further having an antiinflammatory and/or antimicrobial coating (32).

8. The implant (10; 70) according to any one of claims 1 to 7, wherein at least the structure element (28, 30; 76, 78; 84, 86) includes a shape memory material designed to deploy above a predetermined temperature.

9. The implant (10; 70) according to any one of claims 1 to 8, wherein at least the structure element (28, 30; 76, 78; 84, 86) includes a superelastic and/or resorbable material.

10. The implant (70) according to any one of claims 1 to 9, wherein each wall of at least one of the clip(s) has a height decreasing from a rear edge to a front edge of the wall.

11. The implant (10; 70) according to any one of claims 1 to 10, wherein each wall of at least one of the clip(s) has a constant height from a rear edge to a front edge of the wall.

12. The implant (70) according to any one of claims 1 to 11, further including, for each wall of at least one of the clip(s), a structural reinforcement element extending along an edge of this wall.

13. The implant (70) according to claim 12, wherein each structural reinforcement element includes a rod (80, 82, 88, 90) having undulations allowing a compression of this structural reinforcement element.

14. The implant (70) according to any one of claims 1 to 13, wherein the clip(s) comprise two clips intended to grip respectively two biological protuberances (38, 40) present in the cavity (34) in order to attach the implant (70) to both biological protuberances at the same time.

15. The implant (70) according to claim 14, wherein the two clips are located one above the other so as to be able to grip respectively a middle concha (38) and an inferior concha (40) of a nasal cavity (34).
